# EUROPÄISCHE PATENTSCHRIFT

(11) **EP 1 185 634 B1**
(45) Veröffentlichungstag und Bekanntmachung des Hinweises auf die Patenterteilung: **22.01.2014**
(21) Anmeldenummer: 00936749.1
(22) Anmeldetag: 17.05.2000
(51) Int. Cl.: A23K 1/00, A61K 38/00, A23K 1/165, C12N 9/98

(54) **ENZYM-INSTANTFORMULIERUNGEN FÜR DIE TIERERNÄHRUNG**
INSTANT FORMULATIONS OF ENZYMES, USED FOR ANIMAL FEED
FORMULATIONS INSTANTANEES D'ENZYMES POUR L'ALIMENTATION DES ANIMAUX

(30) Priorität: 18.05.1999 DE 19922753
(43) Veröffentlichungstag der Anmeldung: 13.03.2002
(73) Patentinhaber: BASF SE, 67056 Ludwigshafen (DE)
(72) Erfinder: HARZ, Hans-Peter, D-67373 Dudenhofen (DE); BETZ, Roland, D-67150 Niederkirchen (DE); HEINZL, Wolfgang, D-67157 Wachenheim (DE); GAUS, Günter, D-68647 Biblis (DE)
(86) Internationale Anmeldenummer: PCT/EP2000/004475
(87) Internationale Veröffentlichungsnummer: WO 2000/070034

(56) Entgegenhaltungen:
- EP-A- 0 758 018
- WO-A-97/12958
- WO-A-99/32612
- US-A- 5 814 501

## Beschreibung

Die vorliegende Erfindung betrifft neuartige Enzym-Instantformulierungen, Verfahren zu deren Herstellung sowie deren Verwendung zur Tierernährung.

Enzyme finden vielfache Anwendung in der Tierernährung. Enzyme werden mit dem Ziel eingesetzt, die Bioverfügbarkeit bestimmter Nährstoffe zu erhöhen oder die Futterverwertbarkeit zu verbessern. So wird beispielsweise das Enzym Phytase dazu eingesetzt, um in Phytat gebundenen Phosphor freizusetzen und somit dessen Bioverfügbarkeit zu gewährleisten. Weiterhin ist bekannt, NSP (Non Starch Polysaccarids)-Enzyme dem Futter zuzusetzen, um unter anderem die Viskosität des Futters im Verdauungstrakt zu reduzieren und dadurch die Verwertbarkeit des Futters zu verbessern.

Die mit einem Enzympräparat versetzten Futtermittel werden jedoch häufig pelletiert, expandiert oder extrudiert, wodurch auf Grund der dabei auftretenden, relativ hohen Temperaturen, die Enzymaktivität vermindert oder gänzlich vernichtet wird. Um diesen Nachteil zu vermeiden, werden Enzyme in Form von Flüssigformulierungen auf das bereits pelletierte Futtermittel aufgedüst.

Die Verwendung von flüssigen Enzympräparaten besitzt jedoch ebenfalls Nachteile. Auf Grund des hohen Wassergehaltes bzw. der hohen Wasseraktivität sind derartige Enzym-Formulierungen nur sehr schwer zu stabilisieren. Häufig beobachtet man einen starken Aktivitätsverlust, eine zunehmende Keimzahl sowie eine Verschlechterung des Aussehens des Präparates während der Lagerung. Insbesondere auf Grund von Temperaturschwankungen ist häufig eine Ausfällung der Enzyme oder anderer darin enthaltener Proteine zu beobachten. Neben der unzureichenden Lagerstabilität stellt außerdem die geringe Aktivität pro Volumeneinheit bei Flüssigformulierungen einen gravierenden Nachteil dar.

Aus der WO 97/12958 sind Enzym-haltige Mikrogranulate bekannt, welche durch Agglomeration erhalten werden. Sie umfassen ein auf einem Träger aufgetragenes Enzympräparat, und wenigstens ein Bindemittel oder zerfallsbescheunigendes Mittel. Die Granulate sind außerdem polymerbeschichtet und besitzen eine Korngröße von weniger als 0,4 mm. Der Enzymgehalt für eine typische Zusammensetzung beträgt weniger als 40 Gew.-%. Die Herstellung einer Enzym-Instantformulierung, welche insbesondere als leicht zu handhabender Futtermittelzusatz geeignet ist, wird darin nicht beschrieben.

Es ist deshalb Aufgabe eine verbesserte Enzym-Formulierung bereitzustellen, welche die oben beschriebenen Nachteile von Flüssigformulierungen vermeidet. Insbesondere sollten Enzymformulierungen hergestellt werden, die einen hohen Enzymanteil und insbesondere eine hohe Enzym-Aktivität pro Volumeneinheit besitzen, lagerstabil sind und in einfacher Weise auf Futtermittel aufzubringen sind.

Überraschenderweise konnte diese Aufgabe durch Bereitstellung von festen Enzym-Instantformulierungen gelöst werden.

Die erfindungsgemäßen Enzym-Instantpulver besitzen eine Reihe entscheidender Vorteile:
Sie können beim Anwender auf Grund ihrer sehr guten Löslichkeit oder Dispergierbarkeit in wässrigem Medium rasch in eine Flüssigformulierung überführt werden. Die ausgezeichneten Instanteigenschaften garantieren ein rasches Auflösen ohne besonderen technischen Aufwand. Nach ihrem Auflösen in flüssigem Medium, wie Wasser, einer wässrigen Lösung oder Dispersion, können die erfindungsgemäßen Präparate in herkömmlicher Weise auf das Futtermittel aufgebracht werden. Da die erfindungsgemäßen Enzym-Präparate in agglomerierter Form vorliegen sind sie besonders gut handhabbar. Insbesondere besitzen sie eine ausgezeichnete Fließfähigkeit, sind praktisch staubfrei und neigen nicht zum Verklumpen während des Auflösens. Von besonderer Bedeutung ist jedoch, dass sie eine im vergleich zu Flüssigpräparaten deutlich verbesserte Lagerstabilität besitzen. Außerdem besitzen sie pro Volumeneinheit eine relativ hohe enzymatische Aktivität wodurch eine Minimierung von Lager- und Transportkosten erreicht wird.

Die erfindungsgemäßen Enzym-Instantformulierungen werden in neuer Art und Weise durch Agglomeration hergestellt.

Ein erster Gegenstand der Erfindung ist somit ein Verfahren zur Herstellung einer Enzym-Instantformulierung, dadurch gekennzeichnet, dass man
a) ein pulverförmiges Material vorlegt, das ausgewählt ist unter
   i) einem in wässrigem Medium löslichen Enzympräparat, erhältlich durch Sprühtrocknung einer Enzym-haltigen Lösung; und
   ii) Gemischen von i) und einem in wässrigem Medium löslichen oder dispergierbaren anorganischen oder organischen Träger;
      und
b) das pulverförmige Material durch gleichzeitiges oder zeitlich versetztes Aufsprühen eines oder mehrerer Sprühmedien zu einem Instantpulver agglomeriert, wobei die Sprühmedien ausgewählt sind unter Enzymlösungen, Bindemittellösungen, Bindemitteldispersionen und Bindemittel-haltigen Enzymlösungen;
wobei man bis zu einer mittleren Korngröße von 0,8 bis 8 mm agglomeriert, und die erhaltene Instantformulierung einem Proteinanteil von etwa 50-95 Gew.-%, bezogen auf das Gesamtgewicht der Formulierung, aufweist.

Das erfindungsgemäße Verfahren kann sowohl kontinuierlich als auch diskontinuierlich (batchweise) durchgeführt werden.

Eine erste bevorzugte Variante des erfindungsgemäßen Verfahrens betrifft die diskontinuierliche Herstellung eines Instantpulvers:
Man agglomeriert batchweise in der Wirbelschicht unter Verwendung eines pulverförmigen Materials gemäß obiger Definition. Dabei legt man dieses Pulver in einem Wirbelbett vor. Die Verwirbelung erfolgt z.B. durch Zufuhr vorgewärmter Luft. Man sprüht eine Enzym-haltige Lösung in die Wirbelschicht, wodurch man das Pulver mit dieser Lösung benetzt und durch deren Klebeeigenschaften zunehmend agglomeriert. Das Einsprühen in die Wirbelschicht kann von oben (Topspray-Verfahren) öder von unten (Bottomspray-Verfahren) erfolgen. Ist die gewünschte Agglomeratgröße erreicht, bzw. die gewünschte Enzymaktivität bzw. -menge eingestellt, so wird das Produkt aus dem Trockner ausgetragen und z.B. mit Hilfe eines Siebs klassiert.

Um eine möglichst hohe Aktivität im Instantpulver zu erhalten, sollte bei der Batch-Fahrweise die Vorlage an Träger möglichst gering gehalten werden und das Wirbelbett zum Ende des Prozesses möglichst bis zur maximalen Füllhöhe gefahren werden. Die konkrete Fahrweise des Wirbelbettes hängt von der jeweiligen Füllhöhe ab. Mit steigender Füllhöhe während des Prozesses steigen auch die Luftmenge und Sprührate an. Zu Beginn des Prozesses soll vorzugsweise auf eine Mindestmenge an Vorlage geachtet werden, da anderenfalls Probleme, beispielsweise durch Verkleben und Klumpenbildung im Produkt, auftreten können. Die einzelnen Verfahrensparameter müssen während der Prozessdauer vorsichtig an die jeweilige Füllmenge im wirbelbett angepasst werden. Bei zu trockener Fahrweise wird zu wenig agglomeriert, bei zu feuchter Fahrweise verklebt das Produkt stark, woraus Klumpenbildung und Verkleben der Apparatur resultiert.

Das im Wirbelbett vorgelegte Material kann auch ein durch Sprühtrocknung erhaltenes pulverförmiges Enzympräparat sein, welches man gegebenenfalls vor dem Aufspühen der Enzymlösung voragglomeriert.

Die Sprühtrocknung von flüssigen Enzympräparaten kann in herkömmlicher Weise durchgeführt werden. Dazu wird die Enzymlösung zum Zerstäuber im Sprühturm gepumpt. Die Zerstäubung erfolgt z.B. mittels einer Druckdüse (Einstoffdüse), einer Zweistoffdüse oder eines Zentrifugalzerstäubers. Die Trocknung der Tröpfchen erfolgt durch einen in den Sprühtrockner geleiteten Heißluftstrom. Bei Verwendung von Zentrifugalzerstäubern erfolgt die Trocknung vorzugsweise im Gleichstrom. Bei Düsen kann die Trocknung auch im Gegen- oder Mischstrom erfolgen. Das Pulver kann am Turm ausgetragen werden oder es wird mit dem Luftstrom mitgeführt und in einem Zyklon und/oder Filter abgetrennt. Je nach Produkt und Fahrweise kann eine Nachtrocknung erforderlich sein, die in einem internen, an den Sprühtrockner aufgeflanschten oder einem externen Wirbelbett erfolgen kann.

Eine weitere bevorzugte Variante des erfindungsgemäßen Verfahrens betrifft die kontinuierliche Wirbelbettagglomeration ohne Verwendung eines anorganischen oder organischen Trägers durch kontinuierliches Aufsprühen einer Enzymlösung. Dazu wird in einem Wirbelbetttrockner zu Beginn des Verfahrens ein pulverförmiges Material, z.B. durch Sprühtrockung erhaltenes Enzympulver, vorgelegt. Die Verwirbelung erfolgt z.B. durch Zufuhr vorgewärmter Luft. Man sprüht auf die Wirbelschicht eine Enzym-haltige Lösung auf, wodurch man das vorgelegte Pulver mit dieser Lösung benetzt und durch deren Klebeeigenschaften zunehmend agglomeriert. Gleichzeitig wird kontinuierlich oder quasi-kontinuierlich, d.h. intervallweise getaktet, eine Teilmenge Agglomerat aus der Wirbelschicht ausgetragen. Die ausgetragene Menge an Agglomerat entspricht in etwa der über die Sprühdüse zugeführten Enzymmenge, korrigiert um die unterschiedlichen Gehalte an Lösungsmittel in der Sprühlösung bzw. im Agglomerat. Bei dieser Fahrweise ist also nur beim Anfahren des Prozesses einmalig eine Vorlage vorzulegen. Der Austrag wird z.B. mit Hilfe eines Siebs klassiert. Dabei anfallendes Grobgut kann dabei gemahlen und kontinuierlich in das Wirbelbett wieder zurückgeführt werden. Feinanteile, wie z.B. aus der Abluftfilteranlage, können ebenfalls kontinuierlich zurückgeführt werden.

Gemäß einer weiteren Verfahrensvariante erfolgt die Herstellung des erfindungsgemäßen Agglomerats kontinuierlich und zwar unter kontinuierlicher Zuführung einer trockenen pulverförmigen Vorlage, wie z.B. eines Enzympulvers, in den Wirbelbetttrockner.

Dafür eignen sich besonders Wirbelbetttrockner mit mehreren Sprüh- und gegebenenfalls Trockenzonen. In der ersten Zone wird Träger oder trockenes Enzympulver aufgegeben, verwirbelt und Enzymlösung und/oder Bindemittel eingesprüht. Das in dieser Zone gebildete Agglomerat wird in die nächste Zone überführt. In diese und gegebenenfalls in einer oder mehreren weiteren Zonen kann ebenfalls Enzym- und/oder Bindemittellösung gleicher oder unterschiedlicher Zusammensetzung eingesprüht werden. Durch einen für alle Zonen gemeinsamen Zuluftstrom oder getrennte Zuluftströme, die entsprechend erwärmt sind, wird das Wasser der aufgesprühten Enzym- oder Bindemittellösung entzogen. In einer oder mehreren der letzten Zonen kann noch nachgetrocknet werden. Hier befindet sich auch der Produktaustrag. Die Aufarbeitung des Produkts erfolgt wie oben beschrieben.

Eine weitere bevorzugte Verfahrensvariante umfasst eine Sprühtrockung von Enzymlösung gekoppelt mit der anschließenden Agglomeration des sprühgetrockneten Enzympulvers. Diese kann diskontinuierlich oder kontinuierlich durchgeführt werden. Bevorzugt ist die kontinuierliche Fahrweise.

Derartige Verfahren können unter Verwendung herkömmlicher Sprühtrocknungsanlagen durchgeführt werden. Vorteilhafterweise erfolgt die Durchführung aber in Vorrichtungen, welche als FSD (Fluidized Spray Dryer) SBD (Spray Bed Dryer) oder MSD (Multi Stage Dryer) bekannt sind.

Von Vorteil ist bei dieser Verfahrensvariante, wenn größere Partikel durch Sprühtrocknung erzeugt werden (mittlerer Durchmesser >80µm). Der anfallende Feinanteil des Pulvers kann bereits im Sprühtrockner in den Prozeß wieder eingebunden werden, wenn man diesen, z.B. nach Abscheidung in einem Zyklon oder Filter, wieder in die feuchte Zone des Trockners zurückführt. Die eigentliche Agglomeration findet dann in einer weiteren Stufe in einem Wirbelbett statt. Diese Stufe kann in den Sprühtrockner integriert sein (internes Wirbelbett) oder sie kann in einer separaten Apparatur (zusätzliches Wirbelbett) durchgeführt werden. In das Wirbelbett wird bei gleichzeitigem Trocknen eine Enzymlösung, eine Enzymlösung welche außerdem Bindemittel enthält oder nur Bindemittel in gelöster oder dispergierter Form eingedüst. Das durch die Sprühtrocknung hergestellte Pulver enthält ein Enzym, das vorzugsweise mit dem aufgesprühten Enzym identisch ist. Die Zusammensetzung und Menge der eingedüsten Flussigkeiten richten sich nach den Klebeeigenschaften der eingesprühten Lösung, der zu erzielenden Agglomeratgrösse und den Prozessbedingungen. Bezogen auf das zu agglomerierende Pulver kann die Menge an Sprühlösung, d.h. die Menge an eingedüster Flüssigkeit, etwa 10 bis 200 % betragen. Je nach aufgesprühter Menge kann eine Nachtrocknung in einer weiteren Stufe erforderlich sein. Die Aufarbeitung des Produkts erfolgt dann in der oben beschriebenen Weise.

Weitere bevorzugte Verfahrensparameter des erfindungsgemäßen Verfahrens:
Auf Grund der in der Regel hohen Temperaturlabilität der aufgespühten Enzyme ist während der erfindungsgemäßen Verfahren die Regelung der Produkttemperatur von besonderer Bedeutung. Sie sollte möglichst niedrig gewählt werden, da mit zunehmender Temperatur und/oder Dauer des Sprühtrocknungs- und Agglomerationsverfahrens die Aktivitätsverluste zunehmen. Typischerweise liegt die Produkttemperatur bei Sprühtrocknung, d.h. die Temperatur des festen sprühgetrockneten Pulvers, bei etwa 50 bis 75 °C, insbesondere bei weniger als etwa 70 °C, häufig weniger als 60 °C. Je länger die Verweilzeit im Wirbelbett ist, um so niedriger sollte die Temperatur gewählt werden.

Die Produkttemperatur während der Agglomeration und Trocknung in der Wirbelschicht, d. h. die Temperatur des im Wirbelbett befindlichen Agglomerates, ist wegen der längeren Verweilzeit in der Vorrichtung niedrig zu wählen und liegt bei Werten von etwa 30 als 50 °C, insbesondere weniger als 45 °C und vorzugsweise bei weniger als 40 °C.

Um den Restfeuchigkeitsgehalt weiter zu verringern ist die Durchführung eine Nachtrocknungsschrittes bevorzugt. Auch während der Nachtrocknung sollte die Produkttemperatur in dem oben genannten Bereich und insbesondere bei 50°C oder weniger liegen. Durch die Nachtrocknung wird der Restfeuchtigkeitgehalt in den erfindungsgemäßen Präparaten auf Werte von weniger als etwa 15 Gew.-%, vorzugsweise etwa 2 bis 10 Gew.-% reduziert.

Die Trocknung während der Agglomeration bzw. die Nachtrocknung wird durch Verwendung von vorgewärmter Zuluft erreicht. Die Zulufttemperatur, die je nach gewählter Sollprodukttemperatur, Luftmenge und Sprührate unterschiedlich sein kann, liegt im Allgemeinen in einem Bereich zwischen 30 und 80 °C. Die Nachtrocknung erfolgt bei niedrigerer Temperatur, nämlich im Bereich von etwa 35 bis 55 °C.

Die Dauer der Agglomeration ist ebenfalls von der Größe des gewählten Ansatzes abhängig liegt aber etwa im Bereich von einer bis mehreren Stunden.

Zur weiteren Verbesserung der Produktqualität kann es von Vorteil sein, das anfallende Agglomerat mit einem wasserlöslichen Coating zu versehen. Dadurch wird die Ausbildung eines Produktabriebes und somit die Staubbildung weitgehend verhindert. Auf Grund des allergenen Potentials einiger Enzyme ist eine derartige Maßnahme von besonderem Interesse. Beispiele für geeignete coating-Materialien sind die oben beschriebenen Bindemittel, wie z.B. Hydroxypropylmethylcellulose und Polyvinylpyrrolidon, oder Polyethylenglykole und Blockpolymere von Polyoxyethylen und Polyoxypropylen. Das Coating-Verfahren kann beispielsweise in einem wirbelbett durchgeführt werden, insbesondere dann, wenn zur Auftragung des Coatings eine Lösung oder Suspension der Coating-Materialen, wie z.B. der oben genannten Materialien, verwendet wird. Die verwendeten Lösungen oder Dispersionen weisen einen Gehalt an Coatingmittel im Bereich von etwa 5 bis 50 Gew.-%, bezogen auf das Gesamtgewicht der Lösung oder Dispersion, auf. Bei Verwendung von Schmelzen, wie z.B. Schmelzen von PEG (Polyethylenglykol) oder Blockpolymeren von Polyoxyethylen und Polyoxypropylen, ist die Durchführung des Coatingschritts in einem Wirbelbett oder einem Mischer, wie z.B. einem Pflugscharmischer der Fa. Lödige, zweckmäßig.

Nach dem Aufbringen des Coatings kann ein erneutes Trocknen erforderlich sein. Auch hierbei sollte die Produkttemperatur im Bereich von etwa 30 bis 50 °C liegen.

Der Gewichtsanteil an Coatingmaterial im Endprodukt beträgt etwa 5 bis 20 % bezogen auf das Gesamtgewicht der Instantformulierung.

Der erfindungsgemäß verwendete Vorlage für die Agglomeration kann neben dem Enzympräparat den Träger enthalten, der vorzugsweise unter inerten anorganischen in Wasser löslichen oder dispergierbaren Pulvern, pulverförmigen, in Wasser löslichen oder dispergierbaren organischen Polymeren oder pulverförmigen in Wasser vorzugsweise löslichen Enzympräparaten ausgewählt ist. Ein "inerter" Träger oder eine "inerte" Vorlage darf keine negativen Wechselwirkungen mit dem(den) Enzym(en) der Instantformulierung zeigen, wie z.B. eine irreversible Inhibierung der Enzymaktivität bewirken, und muß für den Einsatz als Hilfsstoff in einem Futtermittelzusatz unbedenklich sein. Außerdem sollte er eine für die Wirbelbettgranulation geeignete Korngrössenverteilung besitzen. Die mittlere Partikelgröße (mittlerer Durchmesser) beträgt etwa 30 bis 300 µm, vorzugsweise etwa 50 bis 200 µm. Grundsätzlich ist die Agglomeration auch mit größeren Partikeln möglich; dies führt jedoch zur Bildung größerer Agglomerate mit längerer Lösezeit.

Als Beispiele für geeignete niedermolekulare anorganische Träger oder Vorlagen sind zu nennen Natriumchlorid, Calciumcarbonat Natriumsulfat und Magnesiumsulfat. Als Beispiele für geeignete organische Pulver sind insbesondere zu nennen Zucker, wie z.B. Glucose, Fructose, Saccharose, sowie Dextrine und Stärkeabbauprodukte. Als Beispiele für organische Polymerträger sind insbesondere zu nennen Stärke- und Cellulosepräparate, insbesondere Maisstärke.

Die erfindungsgemäß zur Sprühtrockung oder Agglomeration eingesetzte Enzymlösung enthält wenigstens ein als Futtermittelzusatz brauchbares Enzym, gelöst in einer wässrigen Phase, wie z.B. keimfreies, entsalztes Wasser. Die Lösung besitzt einen Proteinanteil im Bereich von etwa 1 bis 50 Gew.-%, vorzugsweise etwa 10 bis 35 Gew.-%, bezogen auf das Gesamtgewicht der Lösung. Der pH-Wert liegt allgemein im Bereich von etwa 4 bis 9. Die Lösung kann weitere übliche Zusätze enthalten. Als Beispiele sind zu nennen: Puffer, wie z.B. Phosphatpuffer; Enzymstabilisatoren, wie z.B. Alkali- oder Erdalkalimetallsalze, wie Natrium- oder Magnesiumsulfat; Lösungsvermittler, wie z.B. Ethanol oder oberflächenaktive Mittel und dergleichen.

Für den Fall, dass die Klebeeigenschaften der aufgesprühten Enzymlösung und des vorgelegten pulverförmigen Trägers nicht ausreichen, um nach dem Aufsprühen ein stabiles Verkleben der Partikel zu gewährleisten, ist zusätzlich die Verwendung eines Bindemittels von Vorteil. Dadurch wird vermieden, dass die Agglomerate beim Trocknen wieder zerfallen. In solchen Fällen ist es bevorzugt, ein in wässrigem Medium lösliches oder dispergierbares Bindemittel in das Wirbelbett einzusprühen. Das Bindemittel kann entweder in der einzusprühenden Enzymlösung gelöst sein oder getrennt davon, gleichzeitig oder zeitlich versetzt, eingesprüht werden. Als Beispiele für geeignete Bindemittel sind zu nennen Lösungen von Kohlehydraten, wie z.B. Glucose, Saccharaose, Dextrine u.a., Zuckeralkohole, wie z.B. Mannit, oder Polymerlösungen, wie beispielsweise Lösungen von Hydroxypropylmethylcellulose (HPMC), Polyvinylpyrrolidon (PVP), ethoxylierte Cellulose (EC), Ethylcellulose oder Propylcellulose. Durch gezielte Wahl von Menge und Klebeeigenschaften des eingesprühten Bindemittels entstehen Agglomerate unterschiedlicher Größe und Festigkeit.

Wird das Bindemittel im Gemisch mit dem Enzym aufgesprüht, so liegt der Bindemittelanteil gewöhnlich im Bereich von etwa 0,5 bis 20 Gew.-%, vorzugsweise etwa 1 bis 10 Gew.-%, bezogen auf das Gesamtgewicht der Lösung.

wird das Bindemittel als separate Lösung aufgesprüht, so liegt der Bindemittelanteil der Lösung im Bereich von etwa 1 bis 30 Gew.-%, bezogen auf das Gesamtgewicht der Lösung. Das Bindemittel liegt hierbei ebenfalls gelöst in einem wassrigen Medium, vorzugsweise keimfreies, entsalztes Wasser, vor. Übliche Zusätze, wie z.B. Puffer, oder Lösungsvermittler können ebenfalls enthalten sein.

Der Anteil des Bindemittels im Endprodukt beträgt erfindungsgemäß 0 bis etwa 20 Gew.-%, beispielsweise etwa 1 bis 6 Gew.-%. Die optimale Menge ist auch von der Art des gewählten Bindemittels abhängig. Dabei ist darauf zu achten, dass negative Einflüsse auf das Produkt, wie z.B. dessen Lösungseigenschaften, Klarheit der Lösung nach dem Auflösen des Instantpulvers, vermieden werden.

Gegenstand der Erfindung sind außerdem die mit Hilfe der oben beschriebenen verfahren erhältlichen Enzym-Instantformulierungen.

Gegenstand sind insbesondere Enzym-Instantformulierungen, umfassend ein ägglomeriertes pulverförmiges Material, das ausgewählt ist unter
i) einem in wässrigem Medium löslichen Enzympräparat, erhältlich durch Sprühtrocknung einer Enzym-haltigen Lösung; und
ii) Gemischen von i) und einem in wässrigem Medium löslichen oder dispergierbaren anorganischen oder organischen Träger,
wobei diese einem Proteinanteil von etwa 50-95 Gew.-%, bezogen auf das Gesamtgewicht der Formulierung aufweist und deren Agglomeratpartikel eine mittlere Korngröße von 0,8 bis 8 mm aufweisen.

Dieses pulverförmige Material ist unter Verwendung eines agglomerierenden Mittels, vorzugsweise einem Enzym, einem Bindemittel oder einem Enzym-Bindmittelgemisch agglomeriert worden.

vorzugsweise werden Enzym-Instantformulierung bereitgestellt, deren Agglomeratpartikel eine mittlere Korngröße von bis zu etwa 6 mm, insbesondere bis 3 mm aufweisen.

Außerdem weisen die erfindungsgemäßen Formulierungen einen hohen Anteil an enzymatisch aktiven Protein auf.

Die erfindungsgemäßen Instantformulierungen enthalten wenigsten ein Enzym, das ausgewählt ist unter Oxidoreduktasen, Transferasen, Lyasen, Isomerasen, Ligasen und Hydrolasen. Beispiele für Hydrolasen, d.h. Enzyme, welche eine hydrolytische Spaltung von chemischen Bindungen bewirken, sind Esterasen, Glycosidasen, Etherhydrolasen, Proteasen, Amidasen, Aminidasen, Nitrilasen und Phosphatasen. Glycosidasen umfassen sowohl Endo- als auch Exo-Glucosidasen, die sowohl α- als auch ß-glycosidische Bindungen spalten. Typische Beispiele hierfür sind Amylasen, Maltasen, Zellulasen, Endo-Xylanasen, ß-Glucanasen, Mannanasen, Lysozyme, Galaktosidasen, ß-Glucuronidasen und dergleichen. Bevorzugt sind insbesondere Nichtstärkepolysaccharid-spaltende Enzyme, wie z.B. Amylase, Glucanase, und Xylanase, sowie Phosphatasen, wie insbesondere Phytase. Besonders bevorzugte Enzym-Instantformulierungen enthalten 1 x 10⁴ bis 1 x 10⁵ U Phytase pro Gramm Gesamtgewicht der Formulierung. 1 U Phytase ist dabei definiert als die Freisetzung von 1 Mikromol anorganischem Phosphat pro Minute aus einem Überschuß an Phytat.

Je nach Fahrweise des erfindungsgemäßen Herstellungsverfahrens sind die Instantformulierungen unterschiedlich aufgebaut. Einige typische Strukturen seien beispielhaft genannt:
- Agglomerat von Partikeln eines anorganischen oder organischen Trägers, agglomeriert mit wenigstens einem Enzym, gegebenenfalls versehen mit einem Coating.
- Agglomerat von Partikeln eines anorganischen oder organischen Trägers, agglomeriert mit wenigstens einem Enzym/Bindemittelgemisch, gegebenenfalls versehen mit einem Coating.
- Agglomerat von Partikeln eines trockenen Enzympräparats, agglomeriert mit wenistens einem gleichen oder verschiedenen Enzym, gegebenenfalls versehen mit einem Coating.
- Agglomerat von Partikeln eines trockenen Enzympräparats, agglomeriert mit einem Gemisch auswenigstens einem Bindemittel und wenigstens einem gleichen oder verschiedenen Enzym, gegebenenfalls versehen mit einem Coating.
- Agglomerat von Partikeln eines trockenen Enzympräparats, agglomeriert mit wenigstens einem Bindemittel, gegebenenfalls versehen mit einem Coating.

Gewünschtenfalls können in den erfindungsgemäßen Trockenprodukten weitere Bestandteile, wie nahrungsrelevante Zusätze, z.B. Vitamine, Aminosäuren, Spurenelemente, oder Konservierungsstoffe enthalten sein.

Gegenstand der Erfindung sind auch Enzym-Instantformulierung des oben beschriebenen Typs, portionsweise verpackt in löslichen Folienbeuteln. Die Portionsgröße ist grundsätzlich frei wählbar, liegt aber im Allgemeinen im Bereich von etwa 100 g bis 2000 g. Die zur Portionierung verwendbaren wasserlöslichen Folien sind aus dem Stand der Technik bekannt und frei im Handel erhältlich. Vorzugsweise verwendet man Polyvinylalkoholfolien, die in unterschiedlichen Qualitäten beispielsweise unter den Handelsnamen Hydrosol® oder Solublon® vertrieben werden. Die Auflösezeit liegt bei derartigen Folien in der Größenordnung von etwa 3 Minuten. Die Foliendicke richtet sich nach der Portionsgröße. Eine 30 µm-Folie kann z.B. für die Verpakung einer 500 g-Portion verwendet werden. Ein oder mehrere Folienbeutel können zweckmäßigerweise mit einer aluminiumkaschierten Umverpackung versehen werden, um Feuchtigkeit von der Folie abzuhalten.

Ein weiterer Gegenstand der Erfindung sind Futtermittelzusammensetzungen, welche neben üblichen Futterbestandteilen wenigstens eine erfindungsgemäße Enzym-Instantformulierung als Beimischung umfassen. Gegenstand der Erfindung sind auch Futtermittelzusammensetzung, auf welche wenigstens eine der oben beschriebenen Enzym-Instantformulierungen nach Auflösen oder Dispergieren in einer wässrigen Phase aufgebracht wurde.

Ein weiterer Gegenstand der vorliegenden Erfindung betrifft die Verwendung erfindungsgemäßer Enzym-Instantformulierungen als Futterzusatz.

Die vorliegende Erfindung wird nun an Hand der folgenden nicht limitierenden Beispiele und der beiliegenden Figuren näher erläutert.

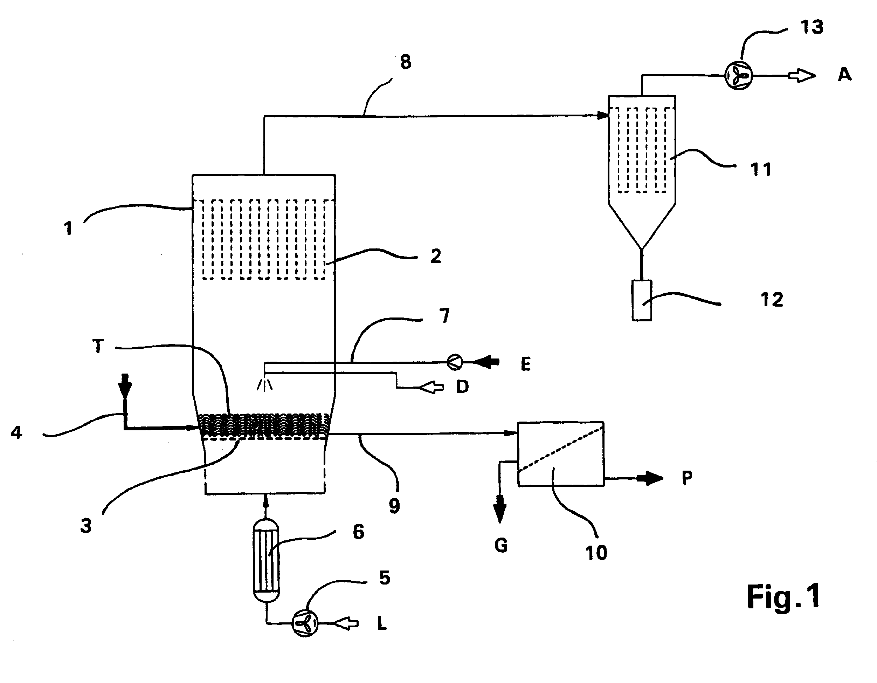

In **Figur 1** ist schematisch eine Wirbelbetttrockner-Anlage zur diskontinuierlichen Herstellung eines geträgerten Enzym-Instantpulvers gezeigt. In einen Wirbelbetttrockner 1, versehen mit einem Innenfilter 2 und einem Lochboden 3, wird von unten mit Hilfe des Gebläßes 5 Luft L, die durch die Heizung 6 vorgewärmt wurde, eingeleitet. Über die Produktaufgabe 4 wird eine Füllung Trägermaterial T zu Beginn des Prozesses in den Wirbelbetttrockner 1 vorgelegt und durch die vorgewärmte Luft verwirbelt. Auf das so gebildete Wirbelbett wird Enzymlösung E, welche gegebenenfalls mit Binder versetzt ist, über die Sprühvorrichtung 7 mit Hilfe von Druckluft D in den Wirbelbetttrockner oberhalb des Wirbelbettes eingesprüht. Sobald die gewünschte Menge eingesprüht ist, wird die Zufuhr von Enzymlösung E unterbrochen, das Rohagglomerat gegebenenfalls im Wirbelbett nachgetrocknet und über den Produktaustrag 9 aus dem Wirbelbetttrockner 1 in die Siebvorrichtung 10 geleitet, wo es in Grobgut G und Produkt P mit der gewünschten Korngröße aufgetrennt wird. Die Prozeßabluft A wird nach Durchtritt durch den Innenfilter 2 über die Ableitung 8 zum Abluftfilter 11 geleitet, dort von Feinstteilen befreit, welche im Behälter 12 gesammelt werden, und wird über das Gebläse 13 aus der Anlage entfernt.

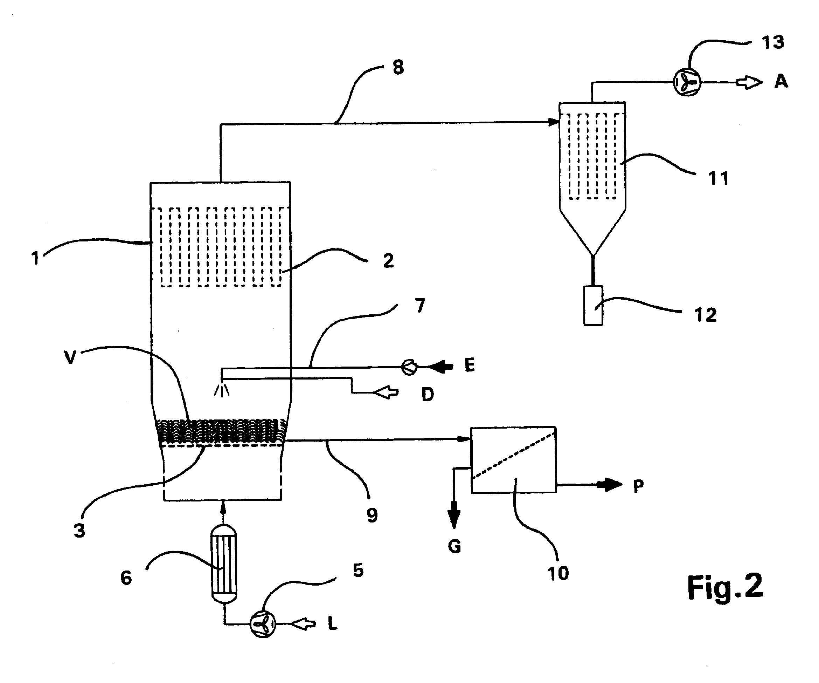

**Figur 2** zeigt eine Abwandlung der Wirbelbettanlage gemäß Figur 1, welche für den kontinuierlichen Betrieb ausgelegt ist. Im Unterschied zu der Vorrichtung gemäß Figur 1 wird in den Wirbelbetttrockner 1 zu Beginn des kontinuierlichen Herstellungsprozesses Trägermaterial V vorgelegt. Hier handelt es sich vorzugsweise um ein bereits getrocknetes Enzympulver, welches anschließend, wie für Figur 1 beschrieben, durch Einblasen von vorgewärmter Luft verwirbelt und mit Enzymlösung E besprüht und agglomeriert wird. Im Unterschied zu der in Figur 1 dargestellten Verfahrensweise wird hier über die Ableitung 9 das gebildete Agglomerat nicht diskontinuierlich, sondern entsprechend der eingesprühten Menge an Enzym kontinuierlich ausgetragen und wie oben beschrieben im Sieb 10 aufgearbeitet.

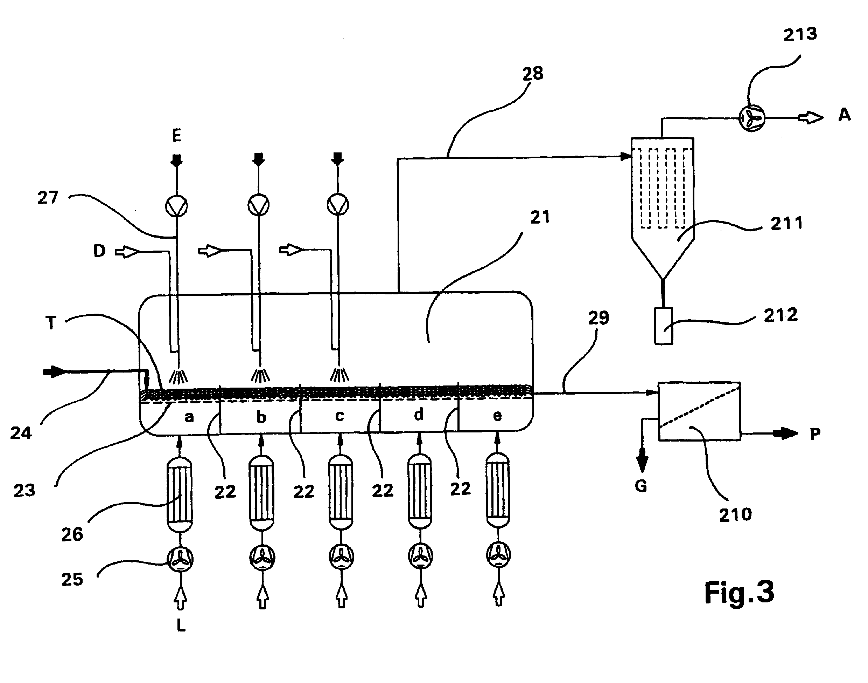

**Figur 3** zeigt eine mehrstufige Wirbelbettanlage zur kontinuierlichen Herstellung eines erfindungsgemäßen Instantpulvers. Der Wirbelbetttrockner 21, ausgestattet mit einem Lochboden 23, wird in seinem Bodenbereich mit Hilfe vertikaler, über die Bodenoberseite hinausragende Trennwände 22 in die Zonen a, b, c, d und e aufgeteilt. Den Sprühzonen a, b und c sind drei identische Sprühvorrichtungen 27 zugeordnet, welche von oben in den Wirbelbetttrockner ragen und über welche mit Hilfe von Druckluft D Enzymlösungen E gleicher oder verschiedener Zusammensetzung eingesprüht werden. Auf die Sprühzonen folgen die Trocknungszonen d und e. Die Zonen a, b, c, d und e sind nach oben hin offen ausgebildet, wobei über den Lochboden 23 hinausragenden Trennwände die die einzelnen Zonen des Wirbelbettes definieren. Die Trennwände können als Überlauf- oder Unterlaufwehre ausgebildet sein. Trägermaterial T, welches entweder ein anorganisches oder organisches Pulver oder ein sprühgetrocknetes Enzympulver sein kann, wird über die Zuleitung 24 in die Zone a eingespeist und dort verwirbelt. Die Verwirbelung erfolgt mit Hilfe von Luft L, welche über ein Gebläse 25 angesaugt, in der Heizung 26 vorgewärmt und von unten in die Zone a eingeleitet wird. Durch Aufsprühen der Enzymlösung E erfolgt eine Agglomeration in der Zone a. Ebenso wird in den Zonen b und c Enzymlösung E eingesprüht. Die Verwirbelung erfolgt auch in diesen Zonen mit entsprechend vorgewärmter Luft, wobei auch hier weiter agglomeriert wird. Ein Teil des Wassers wird, wie auch in Zone a, durch die erwärmte Luft entzogen. Die Zonen d und e sind als reine Trocknungszonen ausgebildet. Das fertige Agglomerat wird aus der Zone e über die Ableitung 29 ausgetragen und mit Hilfe des Siebs 210 in Grobgut G und Produkt P mit der gewünschten Korngröße getrennt. Abluft A wird aus dem Trockner 21 über die Leitung 28 abgeleitet, im Abluftfilter 211 von Feinteilen befreit und über das Gebläse 213 aus dem Prozeß entfernt. Die abgetrennten Feinteile werden im Behälter 212 gesammelt.

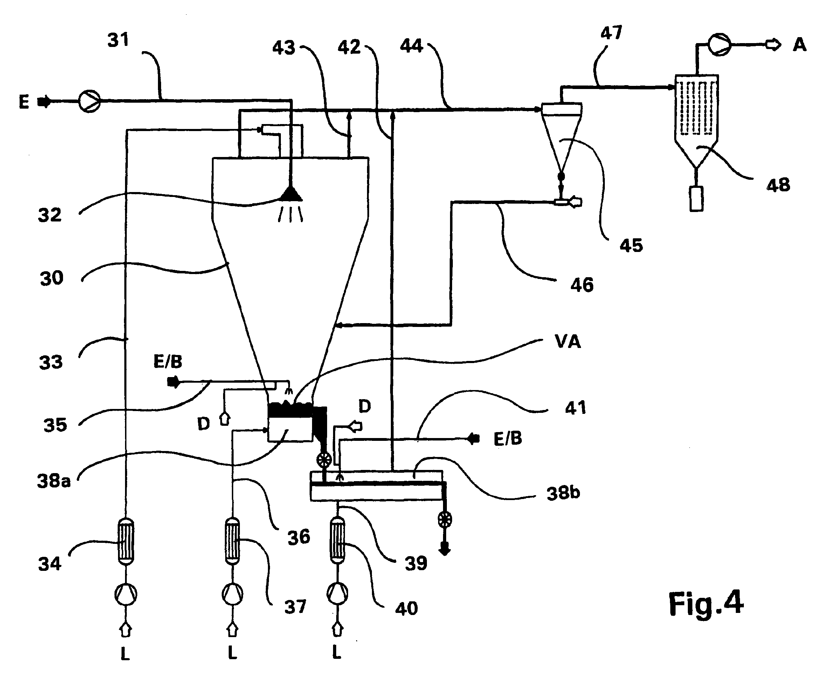

Figur 4 zeigt eine schematische Darstellung einer Fluidized Spray Dryer (FSD)-Trocknungsanlage zur kontinuierlichen Herstellung eines erfindungsgemäßen Enzym-Instantpulvers. Hierbei wird Enzymlösung E über die Zuleitung 31 in den Kopf des FSD-Trockners 30 eingeleitet und mit Hilfe des Zerstäubers 32 zerstäubt. Die Trocknung erfolgt durch Einleitung von Luft im Gleichstrom über das Leitungssystem 33. Die Luft wird dabei über die Heizung 34 vorgewärmt. Das sprühgetrocknete Enzympulver sammelt sich im integrierten Wirbelbett 38a im Boden des FSD-Trockners 30 und wird dort mit Hilfe einer Sprühvorrichtung 35 unter Verwendung von Druckluft D mit Enzymlösung E und/oder Bindemittel B besprüht und mit eingeleiteter Luft verwirbelt wird. Die Luft wird dazu mit Hilfe der Heizung 37 vorgewärmt und durch die Zuleitung 36 unterhalb des Anströmbodens des integrierten Wirbelbettes 38a zugeführt. Das dabei anfallende Voragglomerat VA gelangt anschließend in ein nachgeschaltetes, externes Wirbelbett 38b. In das Wirbelbett 38b wird von unten über die Zuleitung 39 Luft eingeführt, welche zuvor über die Heizung 40 vorgewärmt wurde. Das im Wirbelbett vorgelegte Voragglomerat VA wird mit Hilfe der Sprühvorrichtung 41 unter Verwendung von Druckluft D erneut mit Enzymlösung E und/oder Bindemittel B besprüht und zum Endprodukt agglomeriert. Das fertige Agglomerat wird aus dem Wirbelbett ausgetragen und kann, wie oben beschrieben, weiter aufgearbeitet werden (nicht gezeigt). Abluft aus dem Wirbelbett 38b und dem FSD-Trockner 30 wird über das Leitungssystem 42, 43, 44 zum Zyklon 45 geleitet und dort von Feingut getrennt, welches über die Leitung 46 in den Trockner 30 zurückgeführt wird. Die vorgereinigte Abluft wird über die Leitung 47 nach Durchlaufen des Filters 48 aus dem Prozeß entfernt.

Die mit Hilfe von Vorrichtungen gemäß den Figuren 1 bis 4 hergestellten erfindungsgemäßen Instantprodukte können in einem weiteren Verfahrensschritt mit einem zusätzlichen Coating versehen werden. Die dazu verwendbare Vorrichtung ist praktisch mit der in Figur 1 gezeigten, diskontinuierlich betriebenen Anlage identisch. Anstelle von Trägermaterial T wird hierzu Enzymagglomerat in den Wirbelbetttrockner 1 eingebracht, mit Hilfe von vorgewärmter Luft verwirbelt und gleichzeitig temperiert. Anstelle von Enzymlösung E wird über die Sprühvorrichtung 7 eine Coating-Lösung aufgesprüht. Das so beschichtete Agglomerat wird, gegebenenfalls nach Nachtrocknung und Kühlen, aus dem Wirbelbetttrockner 1 entfernt und im Sieb 10 von Grobgut G befreit. Die Prozeßabluft wird, wie für Figur 1 beschrieben, aufgearbeitet.

### Beispiel 1:

### Agglomeration in der Wirbelschicht mit vorgelegtem anorganischen Träger (Batchfahrweise)

Als Träger (800 g) wurde eine Siebfraktion von Natriumsulfat (<150 µm, wasserfrei) verwendet. In der Enzymlösung (Phytase; Trockenmasse 25,2 %; 15500 U/g) wurden 20 % MgSO₄*7H₂O aufgelöst. Der Träger wurde im Wirbelbett vorgelegt und die Enzymlösung (7900 g) bis zum Erreichen der gewünschten Endaktivität und Agglomeratgröße aufgesprüht.

Bei dem Versuch wurde ein Laborwirbelbett von Niro-Aeromatic, Typ MP-1, mit großem Plexiglaskonus (Anströmboden Durchmesser 170 mm) und einem Lochboden mit 16 % freier Fläche verwendet.

Es wurde mit einer 1,2 mm Zweistoffdüse gesprüht, die zu Beginn am Konus untere Position, später bei höherer Wirbelschicht am Konus obere Position eingebaut war (Topsprayfahrweise). Die Lösung wurde mit einer Membranpumpe der Fa. ProMinent zudosiert.

Die Sprühdauer betrug ca. 4 h, anschließend wurde ca. 1 h nachgetrocknet. Die zulufttemperatur wurde nach der Produkttemperatur geregelt, die zu Beginn 45°C und nach ca. 1 h 40°C betrug. Die Zulufttemperatur, die je nach Sollprodukttemperatur, Luftmenge und Sprührate unterschiedlich war, betrug während der ersten Stunde zwischen 52 und 56 °C, danach 50 °C, langsam bis Sprühende ansteigend auf 65 °C. Bei der Nachtrocknung fiel die Zulufttemperatur wieder bis auf 42 °C. Die Luftmenge betrug zu Beginn 40 m³/h, wurde dann schrittweise bis Sprühende auf 120 m³/h erhöht. Beim Nachtrocknen wurde mit 80 m³/h gefahren. Die Sprührate betrug zu Beginn ca. 230 g/h, danach wurde sie bis Sprühende schrittweise auf ca. 3500 g/h erhöht. Zu Beginn wurde mit einem Sprühdruck von 1,2 bar gefahren, dann schrittweise bis 2 h Sprühdauer auf 1,8 bar erhöht.

Die Agglomeration begann nach ca 1,5 h und mit weiterer Sprühdauer wurde immer stärker agglomeriert. Der Produktaustrag war grob und relativ gleichmäßig agglomeriert und enthielt kaum Feinanteil. Es wurden 3700 g Produkt mit einer Restfeuchte von 10 % ausgetragen.

Man erhält ein Instantpulver mit folgenden Kenndaten:

| | |
|---|---|
| Restfeuchte | : 6 bis 12 % |
| Aktivität | : 22000 bis 25000 U/g (bei Enzymlösungen (Phytase) mit hoher Aktivität auch größer) |
| Staubverhalten | : visuell nahezu staubfrei |
| Fließverhalten | : visuell gut fließend |
| Aussehen | : grobes, gleichmäßiges und bräunliches Agglomerat, evtl. mit wenigen kleinen Klümpchen, die abgesiebt werden können |
| Mittlerer Teichendurchmesser | : 1900 µm |

### Beispiel 2:

### Agglomeration eines Enzympulvers in der Wirbelschicht mit Bindemittel (Batchfahrweise)

Gegenüber dem Prozeß gemäß Beispiel 1 wurde anstelle des inerten Trägermaterials ein Phytasepulver (600 g; 33000 U/g, Restfeuchte 14 %; gegebenenfalls fein agglomeriert) als Vorlage im Wirbelbett verwendet. Bei dem Enzympulver handelt es sich um ein Trockenpulver, das durch Sprühtrocknung gewonnen wurde. Als Sprühlösung wurde eine Phytaselösung (600 g; Trockenmasse 25,2 %; 15500 U/g Aktivität) verwendet. Nach dem Aufsprühen der Phytaselösung auf das vorgelegte Phytasepulver wurde sofort auf den feucht agglomerierten Ansatz eine HPMC-Lösung (17g HPMC in 153g vollentsalztem Wasser) als Bindemittel aufgesprüht und das Enzympulver weiter agglomeriert.

Bei dem Versuch wurde ein Laborwirbelbett von Niro-Aeromatic, Typ MP-1, mit großem Plexiglaskonus (Anströmboden Durchmesser 170 mm) und einem Lochboden mit 12 % freier Fläche verwendet. Es wurde mit einer 1,0 mm Zweistoffdüse gesprüht, die am Konus in der unteren Position eingebaut war (Topsprayfahrweise). Die Lösungen wurden mit einer Membranpumpe von ProMinent zudosiert.

Die Sprühdauer für die Enzym-haltige Sprühlösung betrug 30 min (Sprührate ca. 1500 g/h), für die Bindemittel-haltige Sprühlösung 26 min (Sprührate ca. 1700 g/h). Anschließend wurde 30 min nachgetrocknet und 26 min abgekühlt. Die Zulufttemperatur wurde nach der Produkttemperatur mit einem Sollwert von 40°C geregelt. Die Zulufttemperatur, die je nach Luftmenge und Sprührate unterschiedlich war, betrug während der Sprühung 80 bis 85°C. Bei der Nachtrocknung wurde die Zulufttemperatur bis auf 41°C gesenkt. Die Luftmenge betrug zu Beginn 25 m³/h, wurde dann schrittweise, entsprechend der Zunahme der Partikelgröße, bis Sprühende auf 70 m³/h erhöht und betrug beim Nachtrocknen und Kühlen 25 m³/h. Es wurde mit einem Sprühdruck von 2,5 bar gefahren.

Das Enzympulver war nach Sprühende der Enzymlösung leicht agglomeriert und wurde durch Aufsprühen der Bindemittellösung weiter agglomeriert. Durch das Bindemittel wurden die Agglomerate zusätzlich stabilisiert, so dass diese nach der Trocknung erhalten blieben. Unter dem Mikroskop sahen die Agglomerate fest verklebt und gleichmäßig aus. Es wurden 840 g Produkt mit einer Restfeuchte von 9 % ausgetragen.

Bei dem Prozeß erhält man ein Instantpulver mit folgenden Kenndaten:

| | |
|---|---|
| Restfeuchte | : 6 bis 13 % |
| Aktivität | : 30000 bis 35000 U/g (bei Enzymlösung mit hoher Aktivität auch größer) |
| Staubverhalten | : visuell nahezu staubfrei |
| Fließverhalten | : visuell gut fließend |
| Aussehen | : grobes, gleichmäßiges und bräunliche Agglomerat, evtl. mit wenigen kleinen Klumpen, die abgesiebt werden können |
| Mittlerer Teichendurchmesser | : 700 µm |

### Beispiel 3:

### Coating eines Instantpulvers in der Wirbelschicht (Batchfahrweise)

Auf das gemäß Beispiel 1 erhaltene Instantpulver (1500 g), Klümpchen wurden zuvor ausgesiebt, wurde eine 20 %ige Lutrol F68-Lösung (395 g; Polyoxyethylen-polyoxypropylen-Blockpolymer) aufgesprüht. Das Endprodukt enthält eine Coatingmenge von ca. 5 %. Das Coating wurde in der gleichen Apparatur mit gleichem Aufbau wie in Beispiel 1 durchgeführt. Die zweistoffdüse war am Konus in der unteren Position eingebaut. Die Sprühdauer betrug ca. 45 min, anschließend wurde ca. 55 min nachgetrocknet. Die Zulufttemperatur wurde nach der Produkttemperatur mit einem Sollwert von 40 °C geregelt. Die Zulufttemperatur betrug je nach Luftmenge und Sprührate zwischen 45 und 50°C. Während der Nachtrocknung fiel die Zulufttemperatur auf 40°C. Die Luftmenge wurde zu Beginn mit 80 bis 100 m³/h gefahren. Beim Nachtrocknen betrug die Luftmenge 80 m³/h. Die Sprührate betrug ca. 530 g/h und der Sprühdruck 1,5 bar.

Das Produkt zeigte ein grobes und gleichmäßiges Aussehen, war durch das Aufsprühen noch etwas weiter agglomeriert und enthielt wenige Klumpen. Unter dem Mikroskop war eine glatte und geschlossene Coatingschicht zu erkennen. Es wurden 1544 g Produkt mit einer Restfeuchte von ca. 8 % ausgetragen.

### Beispiel 4:

### Tests zur Beurteilung der Instanteigenschaften

Zur Prüfung der Löseeigenschaften wurde ein Test entwikelt, der die Handbarkeit des Produkts beim Kunden simulieren soll.

Dazu werden in einem 3 Liter Becherglas 1960 g Wasser vorgelegt und mit einem Intensivrührer bei ca. 220 bis 240 U/min gerührt. 40 g des Instantpulvers werden dann schnell (ca. 1 bis 2 Sekunden) eingemischt. Der Ansatz entspricht einer 2 %igen Lösung und, im Falle von Phytase einer Aktivität von 400 bis 600 U/g bei Verwendung eines Instantpulvers mit einer Aktivität von etwa 20000 bis 30000 U/g. Liegt die Aktivität des Instantpulvers außerhalb der üblichen Grenzen, muß der Ansatz auf die jeweilige Aktivität berechnet werden. (Sollaktivität der Lösung bei Phytase - 500 U/g). Bei der Zugabe des Pulvers wird gleichzeitig eine Stopuhr gestartet.

Bei der Beurteilung der Instanteigenschaften wird besonders auf folgende Punkte beachtet:
- Klumpenbildung bei der Zugabe des Instantpulvers
- Zeitpunkt des Zerfallens der Agglomerate
- Zeitpunkt, bei dem das Pulver fast vollständig gelöst ist (es dürfen nur noch wenige große Partikel vorhanden sein)
- Zeitpunkt, bei dem das Pulver vollständig gelöst ist
- Schaumbildung beim Auflösen
- Klarheit der Lösung
- sonstige Beobachtungen

Wird das nach Beispiel 1 hergestellte Instantpulver diesem Test unterworfen, ergibt sich folgendes Bild:
- Keine Klumpenbildung bei Zugabe des Instantpulvers oder während des Lösevorgangs;
- Die Agglomerate zerfallen nach ca. 30 Sekunden;
- Das Instantpulver ist nach ca. 1,5 Minuten bis auf wenige kleine Partikel gelöst;
- Nach insgesamt 2,5 Minuten sind auch die letzten Teilchen gelöst;
- Kein Auftreten von Schaum;
- Nach dem vollständigen Auflösen zeigt die Lösung keine Trübung.

## Patentansprüche

1. Verfahren zur Herstellung einer Enzym-Instantformulierung, **dadurch gekennzeichnet, dass** man
a) ein pulverförmiges Material vorlegt, das ausgewählt ist unter
i) einem in wässrigem Medium löslichen Enzympräparat, erhältlich durch Sprühtrocknung einer Enzym-haltigen Lösung; und
ii) Gemischen von i) und einem in wässrigem Medium löslichen oder dispergierbaren anorganischen oder organischen Träger;
und
b) das pulverförmige Material durch gleichzeitiges oder zeitlich versetztes Aufsprühen eines oder mehrerer Sprühmedien zu einem Instantpulver agglomeriert, wobei die Sprühmedien ausgewählt sind unter Enzymlösungen, Bindemittellösungen, Bindemitteldispersionen und Bindemittel-haltigen Enzymlösungen;
wobei man bis zu einer mittleren Korngröße von 0,8 bis 8 mm agglomeriert, und die erhaltene Instantformulierung einem Proteinanteil von etwa 50-95 Gew.-%, bezogen auf das Gesamtgewicht der Formulierung, aufweist.

2. Verfahren nach Anspruch 1, **dadurch gekennzeichnet, dass** die Agglomeration kontinuierlich oder diskontinuierlich in einem Wirbelbett erfolgt.

3. Verfahren nach einem der vorherigen Ansprüche, **dadurch gekennzeichnet, dass** man bis zu einer mittleren Korngröße von bis zu etwa 6 mm agglomeriert.

4. Verfahren nach Anspruch 1, **dadurch gekennzeichnet, dass** man die Agglomeration des sprühgetrockneten Enzympräparats mittels eines internen in den Sprühtrockner integrierten oder mittels eines externen Wirbelbetts durchführt.

5. Verfahren nach einem der Ansprüche 1 bis 3, **dadurch gekennzeichnet, dass** der in wässrigem Medium lösliche oder dispergierbare Träger ein inertes anorganisches oder organisches niedermolekulares Pulver oder ein pulverförmiges organisches Polymer ist.

6. Verfahren nach einem der vorhergehenden Ansprüche, **dadurch gekennzeichnet, dass** man das Agglomerat auf einen Restfeuchtigkeitsgehalt von weniger als etwa 15 Gew.-% trocknet.

7. Verfahren nach einem der vorhergehenden Ansprüche, **dadurch gekennzeichnet, dass** man die Agglomerat-Partikel mit einem wasserlöslichen Überzug versieht und anschließend gegebenenfalls erneut trocknet.

8. Enzym-Instantformulierung, umfassend ein agglomeriertes pulverförmiges Material, das ausgewählt ist unter
i) einem in wässrigem Medium löslichen Enzympräparat; erhältlich durch Sprühtrocknung einer Enzym-haltigen Lösung; und
ii) Gemischen von i) und einem in wässrigem Medium löslichen oder dispergierbaren anorganischen oder organischen Träger,
wobei diese einem Proteinanteil von etwa 50-95 Gew.-%, bezogen auf das Gesamtgewicht der Formulierung aufweist und deren Agglomeratpartikel eine mittlere Korngröße von 0,8 bis 8 mm aufweisen.

9. Enzym-Instantformulierung nach Anspruch 8,**dadurch gekennzeichnet, dass** das Enzym ausgewählt ist unter Oxidoreduktasen, Transferasen, Lyasen, Isomerasen, Ligasen, Phosphatasen und Hydrolasen.

10. Enzym-Instantformulierung nach Anspruch 9, **dadurch gekennzeichnet, dass** die Hydrolase ein Nichtstärkepolysaccharid-spaltendes Enzym ist.

11. Enzym-Instantformulierung nach Anspruch 10, **dadurch gekennzeichnet, dass** die Phosphatase Phytase ist.

12. Enzym-Instantformulierung nach Anspruch 11, **dadurch gekennzeichnet, dass** sie 1·10⁴ bis 1·10⁵ U Phytase pro Gramm Gesamtgewicht umfasst.

13. Enzym-Instantformulierung nach einem der Ansprüche 8 bis 12, **dadurch gekennzeichnet, dass** die Agglomeratpartikel zusätzlich mit einem wasserlöslichen Überzug versehen sind.

14. Enzym-Instantformulierung nach einem der Ansprüche 8 bis 13, portionsweise verpackt in löslichen Folienbeuteln.

15. Pelletierte Futtermittelzusammensetzung, auf welche wenigstens eine Enzym-Instantformulierung gemäß einem der Ansprüche 8 bis 14 nach Auflösen oder Dispergieren in einer wässrigen Phase aufgebracht wurde.

16. Verwendung einer Enzym-Instantformulierung nach einem der Ansprüche 8 bis 14 oder erhältlich nach einem Verfahren gemäß einem der Ansprüche 1 bis 7 zur Herstellung einer pelletierten Futtermittelzusammensetzung nach Anspruch 15.

## Claims

1. A process for preparing an instant enzyme formulation, which comprises
a) introducing a pulverulent material which is selected from
i) an enzyme preparation which is soluble in aqueous medium, obtainable by spray-drying an enzyme-comprising solution; and
ii) mixtures of i) and an inorganic or organic support which is soluble or dispersible in aqueous medium;
and
b) agglomerating the pulverulent material by simultaneous or time-staggered spraying of one or more spray media to form an instant powder, the spray media being selected from enzyme solutions, binder solutions, binder dispersions and binder-comprising enzyme solutions;
agglomeration being carried out up to a mean particle size of from 0.8 to 8 mm, and the instant formulation obtained having a protein content of about 50-95% by weight, based on the total weight of the formulation.

2. The process according to claim 1 wherein the agglomeration is carried out continuously or batchwise in a fluidized bed.

3. The process according to one of the preceding claims wherein agglomeration is carried out up to a mean particle size of up to about 6 mm.

4. The process according to claim 1 wherein the agglomeration of the spray-dried enzyme preparation is carried out using a fluidized bed which is internally integrated into the spray-dryer or using an external fluidized bed.

5. The process according to one of claims 1 to 3 wherein the support which is soluble or dispersible in aqueous medium is an inert inorganic or organic low-molecular powder or a pulverulent organic polymer.

6. The process according to one of the preceding claims wherein the agglomerate is dried to a residual moisture content of less than about 15% by weight.

7. The process according to one of the preceding claims wherein the agglomerate particles are provided with a water-soluble coating and then if necessary are dried again.

8. An instant enzyme formulation comprising an agglomerated pulverulent material which is selected from
i) an enzyme preparation which is soluble in aqueous medium, obtainable by spray-drying an enzyme-comprising solution; and
ii) mixtures of i) and an inorganic or organic support which is soluble or dispersible in aqueous medium,
said instant enzyme formulation having a protein content of about 50-95% by weight, based on the total weight of the formulation, and its agglomerate particles having a mean particle size of from 0.8 to 8 mm.

9. The instant enzyme formulation according to claim 8 wherein the enzyme is selected from oxidoreductases, transferases, lyases, isomerases, ligases, phosphatases and hydrolases.

10. The instant enzyme formulation according to claim 9 wherein the hydrolase is a non-starch-polysaccharide-cleaving enzyme.

11. The instant enzyme formulation according to claim 10 wherein the phosphatase is phytase.

12. The instant enzyme formulation according to claim 11 wherein it comprises from 1 x 10⁴ to 1 x 10⁵ U of phytase per gram of total weight.

13. The instant enzyme formulation according to one of claims 8 to 12 wherein the agglomerate particles are additionally provided with a water-soluble coating.

14. The instant enzyme formulation according to one of claims 8 to 13 packaged in portions in soluble film pouches.

15. A pelleted feedstuff composition onto which has been applied at least one instant enzyme formulation according to one of claims 8 to 14 after dissolution or dispersion in an aqueous phase.

16. The use of an instant enzyme formulation according to one of claims 8 to 14 or obtainable by a process according to one of claims 1 to 7 for producing a pelleted feedstuff composition according to claim 15.

## Revendications

1. Procédé pour la préparation d'une formulation instantanée d'enzymes, **caractérisé en ce qu'**on
a) dispose au préalable un matériau sous forme de poudre, qui est choisi parmi
i) une préparation enzymatique soluble en milieu aqueux, pouvant être obtenue par séchage par pulvérisation d'une solution contenant des enzymes ; et
ii) les mélanges de i) et d'un support inorganique ou organique soluble ou dispersible en milieu aqueux ; et
b) agglomère le matériau sous forme de poudre par pulvérisation simultanée ou décalée dans le temps d'un ou de plusieurs milieux de pulvérisation en une poudre instantanée, les milieux de pulvérisation étant choisis parmi les solutions enzymatiques, les solutions de liants, les dispersions de liants et les solutions enzymatiques contenant des liants ;
en agglomérant jusqu'à une grosseur moyenne des grains de 0,8 à 8 mm, et la formulation instantanée obtenue présentant une proportion de protéines d'environ 50-95% en poids, par rapport au poids total de la formulation.

2. Procédé selon la revendication 1, **caractérisé en ce que** l'agglomération est réalisée de manière continue ou discontinue dans un lit fluidisé.

3. Procédé selon l'une quelconque des revendications précédentes, **caractérisé en ce qu'**on agglomère jusqu'à une grosseur moyenne de grain allant jusqu'environ 6 mm.

4. Procédé selon la revendication 1, **caractérisé en ce qu'**on réalise l'agglomération de la préparation enzymatique séchée par pulvérisation au moyen d'un lit tourbillonnant interne intégré dans le sécheur par pulvérisation ou au moyen d'un lit tourbillonnant externe.

5. Procédé selon l'une quelconque des revendications 1 à 3, **caractérisé en ce que** le support soluble ou dispersible en milieu aqueux est une poudre de bas poids moléculaire inerte, inorganique ou organique ou un polymère organique sous forme de poudre.

6. Procédé selon l'une quelconque des revendications précédentes, **caractérisé en ce qu'**on sèche l'agglomérat jusqu'à une teneur résiduelle en humidité inférieure à environ 15% en poids.

7. Procédé selon l'une quelconque des revendications précédentes, **caractérisé en ce qu'**on apprête les particules d'agglomérat avec un revêtement soluble dans l'eau puis on les sèche le cas échéant à nouveau.

8. Formulation instantanée d'enzymes, comprenant un matériau aggloméré sous forme de poudre, qui est choisi parmi
i) une préparation enzymatique soluble en milieu aqueux, pouvant être obtenue par séchage par pulvérisation d'une solution contenant des enzymes ; et
ii) les mélanges de i) et d'un support inorganique ou organique soluble ou dispersible en milieu aqueux,
présentant une proportion de protéines d'environ 50-95% en poids, par rapport au poids total de la formulation et dont les particules d'agglomérat présentent une grosseur moyenne des grains de 0,8 à 8 mm.

9. Formulation instantanée d'enzymes selon la revendication 8, **caractérisée en ce que** l'enzyme est choisie parmi les oxydo-réductases, les transférases, les lyases, les isomérases, les ligases, les phosphatases et les hydrolases.

10. Formulation instantanée d'enzymes selon la revendication 9, **caractérisée en ce que** l'hydrolase est une enzyme dissociant les polysaccharides non amidon.

11. Formulation instantanée d'enzymes selon la revendication 10, **caractérisée en ce que** la phosphatase est une phytase.

12. Formulation instantanée d'enzymes selon la revendication 11, **caractérisée en ce qu'**elle comprend 1•10⁴ à 1•10⁵ U de phytase par gramme de poids total.

13. Formulation instantanée d'enzymes selon l'une quelconque des revendications 8 à 12, **caractérisée en ce que** les particules d'agglomérat sont en plus apprêtées avec un revêtement soluble dans l'eau.

14. Formulation instantanée d'enzymes selon l'une quelconque des revendications 8 à 13, emballée par portions dans des sachets en feuille solubles dans l'eau.

15. Composition pelletisée d'aliment pour animaux, sur laquelle a été appliquée au moins une formulation instantanée d'enzymes selon l'une quelconque des revendications 8 à 14 après dissolution ou dispersion dans une phase aqueuse.

16. Utilisation d'une formulation instantanée enzymatique selon l'une quelconque des revendications 8 à 14 ou pouvant être obtenue selon un procédé selon l'une quelconque des revendications 1 à 7 pour la préparation d'une composition pelletisée d'aliment pour animaux selon la revendication 15.
